# EUROPÄISCHE PATENTSCHRIFT

(11) **EP 3 248 009 B1**
(45) Veröffentlichungstag und Bekanntmachung des Hinweises auf die Patenterteilung: **01.09.2021**
(21) Anmeldenummer: 16703069.1
(22) Anmeldetag: 20.01.2016
(51) Int. Cl.: G01N 33/543, A61B 10/00, A61B 10/02

(54) **VORRICHTUNG ZUR IN-VIVO UND/ODER IN-VITRO ANREICHERUNG VON ZIELSTRUKTUREN IN EINER PROBEFLÜSSIGKEIT UND VERFAHREN ZU DEREN HERSTELLUNG**
DEVICE FOR THE IN-VIVO AND/OR IN-VITRO ENRICHMENT OF TARGET STRUCTURES IN A SAMPLE LIQUID AND METHOD FOR THE PRODUCTION THEREOF
DISPOSITIF D'ENRICHISSEMENT, IN-VIVO ET/OU IN VITRO, DE STRUCTURES CIBLES DANS UN ÉCHANTILLON LIQUIDE ET PROCÉDÉ POUR SA FABRICATION

(30) Priorität: 20.01.2015 DE 102015200876
(43) Veröffentlichungstag der Anmeldung: 29.11.2017
(73) Patentinhaber: GILUPI GmbH, 14473 Potsdam (DE)
(72) Erfinder: NIESTROJ, Robert, 14473 Potsdam (DE); LÜCKE, Klaus, 14542 Werder/Havel (DE); KRUSEKOPF, Solveigh, 14473 Potsdam (DE); SCHERAG, Frank, Daniel, 79106 Freiburg (DE); RÜHE, Jürgen, 79356 Eichstetten (DE); BRANDSTETTER, Thomas, 79110 Freiburg (DE)
(74) Vertreter: Grünecker Patent- und Rechtsanwälte PartG mbB
(86) Internationale Anmeldenummer: PCT/EP2016/051136
(87) Internationale Veröffentlichungsnummer: WO 2016/116503

(56) Entgegenhaltungen:
- EP-A1- 2 807 979
- WO-A1-2010/145824
- CN-A- 103 691 066

## Beschreibung

Die Erfindung betrifft eine Vorrichtung zur in-vivo und/oder in-vitro Anreicherung von Zielstrukturen in einer Probeflüssigkeit, umfassend wenigstens einen Funktionsabschnitt, der mit Rezeptoren zur Anreicherung der Zielstrukturen bestückt ist.

Eine gattungsgemäße Vorrichtung ist aus der WO 2010/145824 A1 bekannt.

Die CN 103691066 A offenbart einen implantierbaren Goldmarker zur Anwendung in der Krebstherapie, der einen zylindrischen oder spiralförmigen Grundkörper umfasst, auf welchem Oligonukleotide immobilisiert werden.

Die EP 2 807 979 A1 betrifft eine Detektionsvorrichtung zur Anreicherung von Probematerial, umfassend eine mit Detektionsrezeptoren bestückte Funktionsfläche, welche an voneinander lösbaren Funktionselementen angeordnet ist. Die Funktionsabschnitte können einzeln lösbar auf einem Führungselement angeordnet werden.

Der Erfindung liegt die Aufgabe zu Grunde, die bekannte Vorrichtung dahingehend zu verbessern, um die Anreicherung der Zielstrukturen in der Probeflüssigkeit zu verbessern.

Zur Lösung der Aufgabe stellt die Erfindung eine Vorrichtung zur in-vivo und/oder in-vitro Anreicherung von Zielstrukturen in einer Probeflüssigkeit bereit, umfassend wenigstens einen Funktionsabschnitt, der mit Rezeptoren zur Anreicherung der Zielstrukturen bestückt ist, wobei der Funktionsabschnitt eine Schraubenform aufweist, die durch Verschraubung eines symmetrischen Ausgangsquerschnitts um eine Verschraubungsachse entsteht, wobei der Ausgangsquerschnitt ein konkaves Polygon ist. Die Vorrichtung wird in eine laminare Strömung wie beispielsweise in ein Blutkreislaufsystem eingebracht und erzeugt hier eine Querströmung, die zu Verwirbelungen der Probeflüssigkeit und der darin enthaltenen Zielstrukturen im Bereich der mit den Rezeptoren bestückten Oberfläche des Funktionsabschnitts führt. Im Ergebnis ist dadurch eine deutlich verbesserte Anreicherung der Zielstrukturen an den Rezeptoren des Funktionsabschnitts möglich. Durch einen Ausgangsquerschnitt, der ein konkaves Polygon ist, kann die Verwirbelung gesteigert und die Oberfläche des Funktionsabschnitts weiter vergrößert werden.

Es kann sich als vorteilhaft erweisen, wenn der Ausgangsquerschnitt wenigstens eines der folgenden Merkmale aufweist:
- Der Ausgangsquerschnitt ist spiegelsymmetrisch, vorzugsweise mehrfach spiegelsymmetrisch.
- Der Ausgangsquerschnitt ist drehsymmetrisch bezüglich einer Drehachse, vorzugsweise mehrzählig drehsymmetrisch, bevorzugt wenigstens zwei-, drei-, vier- oder fünfzählig drehsymmetrisch. Die Drehsymmetrie (oder Radiärsymmetrie) ist eine Form der Symmetrie, bei der die Drehung des Ausgangsquerschnitts um einen gewissen Winkel um eine Drehachse diesen Ausgangsquerschnitt wieder mit sich selbst zur Deckung bringt. Diese Drehachse verläuft vorzugsweise durch den Flächenschwerpunkt des Ausgangsquerschnitts. Man spricht von einer n-zähligen Radiär- oder Drehsymmetrie, wenn eine Drehung um 360°/n den Ausgangsquerschnitt auf sich selbst abbildet. Beispielsweise sind gleichseitige Dreiecke (vgl. erstes Ausgangsbeispiel) dreizählig drehsymmetrisch und besitzen eine Symmetrieachse senkrecht zur Dreiecksebene, die es bei Drehung um 120° und 240° auf sich selbst abbildet.
- Der Ausgangsquerschnitt ist ein Dreieck (Trigon), Viereck (Tetragon), Fünfeck (Pentagon), Sechseck (Hexagon), Siebeneck (Heptagon), Achteck (Oktagon), Neuneck (Nonagon, Enneagon), Zehneck (Dekagon), Elfeck (Hendekagon) oder Zwölfeck (Dodekagon). Derartige Querschnittsformen sind besonders symmetrisch und begünstigen die Strömung in einem Strömungssystem.
- Der Ausgangsquerschnitt ist konkav (mindestens ein Innenwinkel ist größer als 180°), vorzugsweise mehrfach konkav (wenigstens zwei Innenwinkel sind größer als 180°), bevorzugt dreifach, vierfach, fünffach, sechsfach konkav (drei, vier, fünf oder sechs Innenwinkel sind größer als 180°).
- Wenigstens eine Ecke des Ausgangsquerschnitts ist abgerundet. Dies dient dazu, eine Beschädigung des Gefäßes der Probeflüssigkeit oder potentielle Materialschwachstellen zu vermeiden.
- Der Ausgangsquerschnitt wird bei gleichbleibender Querschnittsform entlang der Verschraubungsachse kleiner oder größer.
- Der Ausgangsquerschnitt umfasst wenigstens einen Arm, der sich vorzugsweise ausgehend von der Drehachse entlang einer Linie erstreckt, wobei der Arm bevorzugt wenigstens eine Verdickung und/oder wenigstens eine Verjüngung aufweist, wobei die Linie sich besonders bevorzugt radial zur Drehachse erstreckt oder bezüglich einer Radialen zur Drehachse gekrümmt ist. Ein solcher Arm bildet einen Ausleger, der bei einer Verschraubung um die Verschraubungsachse eine verhältnismäßig große Oberfläche bei vergleichsweise geringer Querschnittsfläche erzeugt. Der Arm ist vorzugsweise am freien Ende abgerundet, um eine Beschädigung des Gefäßes der Probeflüssigkeit zu vermeiden.
- Der Ausgangsquerschnitt entsteht durch nachfolgende Verschneidung der Schraubenform mit einem geometrischen Körper, insbesondere einem bezüglich der Verschraubungsachse rotationssymmetrischen Körper wie beispielsweise einem Kegel. Wenn beispielsweise die Schraubenform nachträglich mit einem koaxialen Kegelmantel verschnitten wird, sodass alle außerhalb des Kegelmantels liegenden Teile der Schraubenform entfernt sind, kann eine räumlich komplexe Form bewerkstelligt werden, bei der der Funktionsabschnitt beispielsweise zu einem Ende hin kleiner oder größer wird, während die Schraubenform im Inneren erhalten bleibt. Eine solche Struktur kann dazu führen, dass die durch den Funktionsabschnitt getrennten Strömungsteile der Probeflüssigkeit stromabwärts des Funktionsabschnitts wieder weitgehend störungsfrei vereint werden.

Es kann ebenfalls von Vorteil sein, wenn die Schraubenform wenigstens eines der folgenden Merkmale aufweist:
- Die Verschraubungsachse ist gleichzeitig die Drehachse. Demnach ist der drehsymmetrische Ausgangsquerschnitt um sich selbst verschraubt. Diese Ausführung begünstigt die Ausbildung laminarer Querströmungen im Strömungssystem der Probeflüssigkeit.
- Die Verschraubungsachse befindet sich innerhalb des Ausgangsquerschnitts, wobei die Verschraubungsachse vorzugsweise durch den Flächenschwerpunkt des Ausgangsquerschnitts verläuft oder außerhalb des Flächenschwerpunkts des Ausgangsquerschnitts verläuft. Die erste bevorzugte Variante, bei der die Verschraubungsachse durch den Flächenschwerpunkt des Ausgangsquerschnitts verläuft, erzeugt vergleichsweise kompakte Verschraubungsstrukturen. Die zweite bevorzugte Variante, bei der die Verschraubungsachse außerhalb des Flächenschwerpunkts des Ausgangsquerschnitts verläuft, erzeugt voluminösere Strukturen mit größerer Oberfläche.
- Die Verschraubungsachse befindet sich außerhalb des Ausgangsquerschnitts. Bei dieser Variante können spiralförmige Verschraubungsstrukturen geschaffen werden, die einen zentralen Strömungsquerschnitt entlang der Verschraubungsachse freihalten.
- Die Ganghöhe beträgt maximal 20 mm, vorzugsweise maximal 5 mm. Die Windungszahl beträgt demnach vorzugsweise 0,2 Windungen pro Millimeter. Bei einer derartigen Ganghöhe bzw. Windungszahl können in einem Blutkreislaufsystem bei den üblichen Strömungsbedingungen ideale Strömungsverhältnisse geschaffen werden.
- Die Schraubenform weist eine konstante Ganghöhe auf. Diese Ausführung begünstigt die Ausbildung einer laminaren Querströmung in einem Strömungssystem und erleichtert die Herstellung der Vorrichtung.
- Die Schraubenform weist eine konstante Steigung auf. Auch diese Ausführung erleichtert die Herstellung der Vorrichtung und kann die Strömungsverhältnisse in einem Strömungssystem weiter verbessern.
- Die Schraubenform weist einen konstanten Radius auf. Diese Ausführung erleichtert ebenfalls die Herstellung und kann für optimale Strömungsverhältnisse in einem Strömungssystem sorgen.

Es kann sich als praktisch erweisen, wenn der Funktionsabschnitt wenigstens eines der folgenden Merkmale aufweist:
- Der Funktionsabschnitt umfasst einen Durchmesser von maximal 3 mm. Der Minimale Durchmesser wird herstellungstechnisch und durch die jeweiligen Materialeigenschaften beschränkt. Derartige Abmessungen sind für Blutgefäße optimiert und beispielsweise durch einen peripheren Venenkatheter in einen Blutkreislauf einbringbar.
- Der Funktionsabschnitt umfasst eine Länge von 10 bis 200 mm. Dieser Längenbereich ist besonders geeignet zur Ausbildung einer mit Rezeptoren bestückten Funktionsfläche.
- Der Funktionsabschnitt befindet sich am führenden bzw. freien (distalen) Ende der Vorrichtung und/oder bildet das führende bzw. freie (distale) Ende der Vorrichtung. Vorzugsweise wird lediglich der Funktionsabschnitt in den Blutkreislauf eines Patienten eingeführt, um unmittelbar in Kontakt mit Blut zu kommen, sodass die Zielstrukturen an den Rezeptoren angereichert werden können.
- Der Funktionsabschnitt besteht aus einem biokompatiblen und leicht zu verarbeiteten Material, beispielsweise Metall, bevorzugt medizinischem Edelstahl, einem optischen oder sonstigen Polymer, beispielsweise Poly-(methylmethacrylat) (PMMA), oder einem keramischen Werkstoff. Die Materialeigenschaften dieser Werkstoffe eignen sich für die Herstellung und erfindungsgemäße Anwendung der Vorrichtung. Der Funktionsabschnitt ist flexibel und/oder elastisch, vorzugsweise biegeelastisch. Derartige Materialeigenschaften sind besonders wünschenswert, um den Funktionsabschnitt durch ein peripheres Venenkatheter-System in den Blutkreislauf eines Patienten einbringen zu können. Durch die Flexibilität und/oder Elastizität kann der Funktionsabschnitt den Verformungen des Blutgefäßes folgen, sodass eine Verletzungsgefahr des Patienten verringert und die Handhabung der Vorrichtung erleichtert wird.
- Der Funktionsabschnitt umfasst eine Beschichtung, die der Kontur des Funktionsabschnitts folgt. Die Beschichtung ist vorzugsweise so dünn, dass die Schraubenform des Funktionsabschnitts durch die Beschichtung nicht nennenswert verändert wird. Demnach weist vorzugsweise auch der beschichtete Funktionsabschnitt dieselbe Schraubenform wie der unbeschichtete Funktionsabschnitt auf.
- Die Beschichtung des Funktionsabschnitts ist biokompatibel und/oder blutabweisend. Eine solche Beschichtung führt dazu, dass mit Ausnahme der erwünschten Zielstrukturen keine Blutbestandteile an der Beschichtung festgehalten werden.
- Die Beschichtung des Funktionsabschnitts ist ein Polymer, vorzugsweise ein Hydrogel, bevorzugt ein vernetztes Hydrogel. Ein Hydrogel weist eine verzweigte Oberflächenstruktur auf, die das Anbinden der gewünschten Rezeptoren sowie das Anbinden der erwünschten Zielstrukturen an den angebundenen Rezeptoren nochmals deutlich verbessern kann.

Ein weiterer Aspekt der vorliegenden Erfindung betrifft die Verwendung der Vorrichtung nach einer der vorangehenden Ausführungen zur in-vitro Anreicherung von Zielstrukturen in einer Probeflüssigkeit, umfassend die Schritte:
- Einbringen des Funktionsabschnitts in eine Probeflüssigkeit mit laminarer Strömung, vorzugsweise in einen Blutkreislauf, so dass sich die Verschraubungsachse entlang oder im Wesentlichen entlang der Strömungsrichtung der Probeflüssigkeit erstreckt. Die Schraubenstruktur des Funktionsabschnitts wirkt als statischer Mixer und verursacht Verwirbelungen, die die Zielstrukturen innerhalb der Probeflüssigkeit durchmischen und die Wahrscheinlichkeit des zufälligen Kontakts mit am Funktionsabschnitt immobilisierten Rezeptoren deutlich erhöht.
- Anreicherung der Zielstrukturen in einer Probeflüssigkeit an den Rezeptoren des Funktionsabschnitts. Diese Anreicherung der Zielstrukturen an den Rezeptoren des Funktionsabschnitts wird durch die schraubenförmige Kontur des Funktionsabschnitts im Strömungssystem aufgrund der genannten Strömungseffekte deutlich erhöht.
- Entnahme des Funktionsabschnitts aus der Probeflüssigkeit. Die Entnahme des Funktionsabschnitts aus der Probeflüssigkeit erfolgt vorzugsweise nach einer Verweildauer von ca. 30 Minuten. Die Analyse der an den Rezeptoren angereicherten Zielstrukturen erfolgt vorzugsweise außerhalb der Probeflüssigkeit.

Die vorliegende Anmeldung betrifft ferner ein Verfahren zur Herstellung der Vorrichtung nach einem der vorangehenden Ansprüche, umfassend die Schritte:
- Bereitstellen eines länglichen Ausgangsmaterials, das über wenigstens einen Teil seiner Länge einen symmetrischen Ausgangsquerschnitt aufweist, wobei das längliche Ausgangsmaterial vorzugsweise ein profilierter Draht ist. Ein Draht mit einem bestimmten Profil (z.B. Flachdraht oder Dreiecksprofil) kann beispielsweise aus Metall im Extrusionsverfahren kostengünstig hergestellt werden. Dieser Profildraht kann als Ausgangsmaterial für die nachfolgende Bearbeitung dienen. Es ist aber ebenfalls möglich einen Profildraht aus Kunststoff oder anderen geeigneten Materialien zu verwenden.
- Erzeugen des Funktionsabschnitts durch Verschrauben zumindest eines Abschnitts des Teils des länglichen Ausgangsmaterials mit dem symmetrischen Ausgangsquerschnitt um eine Verschraubungsachse. Dazu wird vorzugsweise ein Ende des Drahtes eingespannt und ein anderes Ende des Drahtes verdreht. Vorzugsweise fällt die Verschraubungsachse mit dem Flächenschwerpunkt des Ausgangsquerschnitts bzw. der Längsachse des Drahtes zusammen. Alternativ dazu ist es möglich, den Draht um ein zylindrisches oder kegelförmiges Element aufzuwickeln, sodass der Draht eine Helixform annimmt, die durch die Mantelfläche des zylindrischen oder kegelförmigen Elements vorgegeben ist. In diesem Fall befindet sich die Verschraubungsachse außerhalb des Ausgangsquerschnitts.
- Aufbringen einer Beschichtung auf den Funktionsabschnitt. Über die Beschichtung des Funktionsabschnitts lassen sich die Wechselwirkungen mit der Probeflüssigkeit und den darin enthaltenen Zielstrukturen gezielt einstellen. Die Beschichtung kann weniger als eine Lage aufweisen. Vorzugsweise handelt es sich um ein Mehrschichtsystem, wie es beispielsweise in der WO 2010/145824 A1 beschrieben ist.
- Anbinden von Rezeptoren zur Anreicherung der Zielstrukturen an den beschichteten Funktionsabschnitt. Bei den Rezeptoren handelt es sich vorzugsweise um Antikörper, die bevorzugt kovalent an die Beschichtung, insbesondere Hydrogel, gebunden werden. Die Anbindung dieser Rezeptoren an die Beschichtung ist ebenfalls in der WO 2010/145824 A1 detailliert beschrieben.

Das Herstellungsverfahren kann mindestens einen weiteren Verfahrensschritt aufweisen, der zur Herstellung wenigstens eines gegenständlichen Merkmals der Vorrichtung nach einer der obigen Ausführungen führt.

### Kurze Beschreibung der Figuren

Es zeigen:

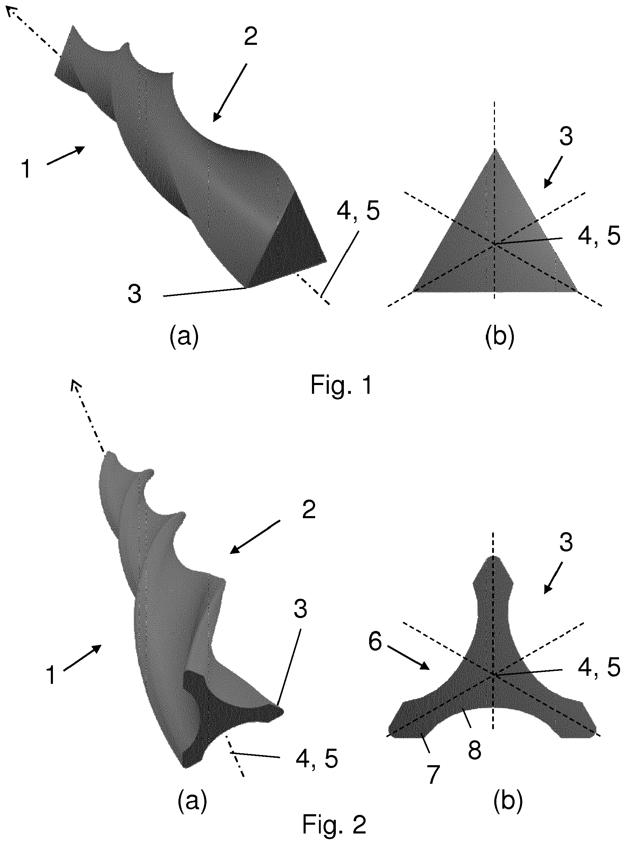

- Figur 1: (a) eine perspektivische Ansicht des Funktionsabschnitts einer Vorrichtung zur in-vivo und/oder in-vitro Anreicherung von Zielstrukturen in einer Probeflüssigkeit, wobei der schraubenförmige Funktionsabschnitt eine Form aufweist, die durch Verschraubung des in (b) gezeigten Ausgangsquerschnitt in Gestalt eines gleichseitigen Dreiecks entlang der Verschraubungsachse erzeugt ist.
- Figur 2: (a) eine perspektivische Ansicht des Funktionsabschnitts einer Vorrichtung zur in-vivo und/oder in-vitro Anreicherung von Zielstrukturen in einer Probeflüssigkeit nach dem ersten Ausführungsbeispiel der Erfindung, wobei der schraubenförmige Funktionsabschnitt eine Form aufweist, die durch Verschraubung des in (b) gezeigten, spiegelsymmetrischen und dreizählig drehsymmetrischen Ausgangsquerschnitts entlang der Verschraubungsachse erzeugt ist.

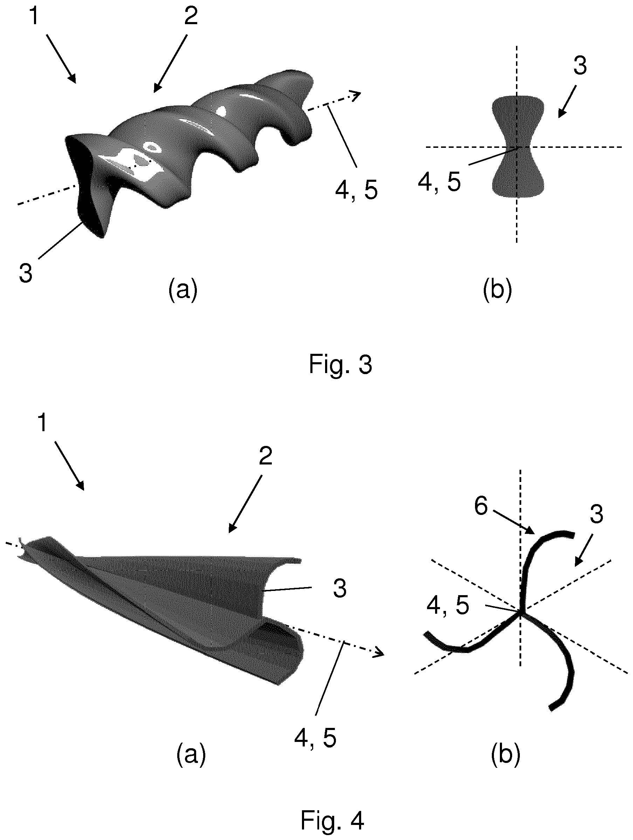

- Figur 3: (a) eine perspektivische Ansicht des Funktionsabschnitts einer Vorrichtung zur in-vivo und/oder in-vitro Anreicherung von Zielstrukturen in einer Probeflüssigkeit nach dem zweiten Ausführungsbeispiel der Erfindung, wobei der schraubenförmige Funktionsabschnitt eine Form aufweist, die durch Verschraubung des in (b) gezeigten, doppelt spiegelsymmetrischen und zweizählig drehsymmetrischen Ausgangsquerschnitts entlang der Verschraubungsachse erzeugt ist.
- Figur 4: (a) eine perspektivische Ansicht des Funktionsabschnitts einer Vorrichtung zur in-vivo und/oder in-vitro Anreicherung von Zielstrukturen in einer Probeflüssigkeit, wobei der schraubenförmige Funktionsabschnitt eine Form aufweist, die durch Verschraubung eines Ausgangsquerschnitts in Gestalt des in (b) gezeigten, dreiarmigen und dreizählig drehsymmetrischen - aber nicht spiegelsymmetrischen - Ausgangsquerschnitts entlang der Verschraubungsachse erzeugt ist.

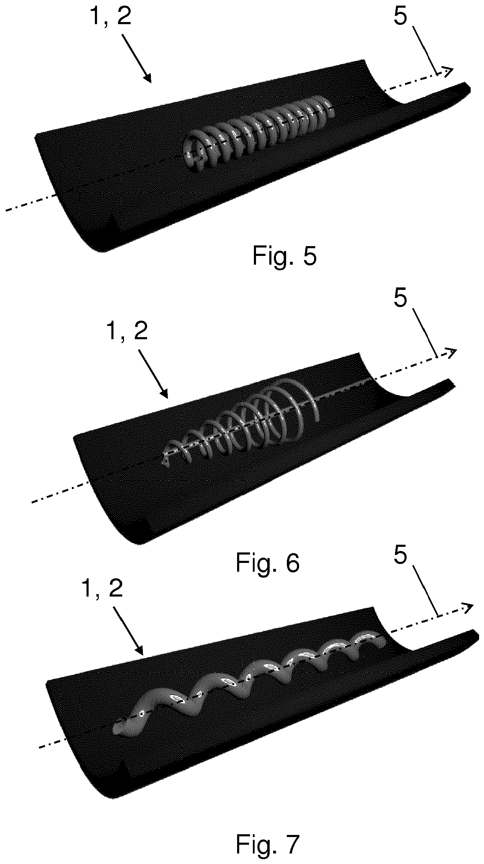

- Figur 5: (auf einer rinnenförmigen Auflage, die nicht zum Erfindungsgegenstand gehört) eine perspektivische Ansicht des Funktionsabschnitts einer Vorrichtung zur invivo und/oder in-vitro Anreicherung von Zielstrukturen in einer Probeflüssigkeit, wobei der Funktionsabschnitt die Form einer zylindrischen Spirale (Helixform) aufweist, die durch Verschraubung eines kreisförmigen Ausgangsquerschnitts entlang einer exzentrischen Verschraubungsachse erzeugt ist, wobei die Spirale eine konstante Steigung und Ganghöhe aufweist.
- Figur 6: (auf einer rinnenförmigen Auflage, die nicht zum Erfindungsgegenstand gehört) eine perspektivische Ansicht des Funktionsabschnitts einer Vorrichtung zur invivo und/oder in-vitro Anreicherung von Zielstrukturen in einer Probeflüssigkeit, wobei der Funktionsabschnitt die Form einer kegelförmigen Spirale aufweist, die durch Verschraubung eines kreisförmigen Ausgangsquerschnitts entlang einer exzentrischen Verschraubungsachse erzeugt ist, wobei die Spirale eine konstante Ganghöhe bei abnehmendem Radius und einer zur Zylinderspitze hin zunehmenden Steigung aufweist.
- Figur 7: (auf einer rinnenförmigen Auflage, die nicht zum Erfindungsgegenstand gehört) eine perspektivische Ansicht des Funktionsabschnitts einer Vorrichtung zur invivo und/oder in-vitro Anreicherung von Zielstrukturen in einer Probeflüssigkeit, wobei der Funktionsabschnitt die Form einer zylindrischen Spirale aufweist, die durch Verschraubung eines kreisförmigen Ausgangsquerschnitts entlang einer exzentrischen Verschraubungsachse erzeugt ist, wobei die zylindrische Spirale im Vergleich zum fünften Ausführungsbeispiel eine größere Ganghöhe und einen kleineren Radius aufweist.

### Detaillierte Beschreibung der bevorzugten Ausführungsbeispiele

Die bevorzugten Ausgangsbeispiele der Erfindung sowie weitere Vorrichtungen werden nachstehend mit Bezug auf die beiliegenden Figuren beschrieben:
Die Erfindung beschreibt eine beispielsweise auf einem Katheter, Stent, Führungsdraht oder dergleichen basierende Vorrichtung zur in vivo Anwendung beim Menschen zur Anreicherung von Zielstrukturen aus Körperflüssigkeiten, insbesondere aus dem Blutkreislauf. Selbstverständlich kann die Vorrichtung auch außerhalb des menschlichen Körpers (in vitro) verwendet werden). Es handelt sich um eine Weiterentwicklung der Erfindung, die in der internationalen Patentanmeldung WO 2010/145824 beschrieben ist. Die Inhalte der WO 2010/145824 A1 sind durch Bezugnahme vollumfänglich hierin enthalten.

Im Rahmen dieser Erfindung wird die in der WO 2010/145824 A1 beschriebene Detektionsvorrichtung um einen anders strukturierten Funktionsabschnitt ergänzt. Die zu Grunde gelegte Struktur der in der WO 2010/145824 A1 beschriebenen Detektionsvorrichtung bleibt dabei erhalten.

Gemäß der vorliegenden Erfindung ist der Funktionsabschnitt ein schraubenförmig gewundenes Element, welches um seine eigene (Torsion), oder in einem definierten Umkreis um eine Achse gewunden sein kann. Es wurde festgestellt, dass in sich verdrehte und um eine Achse gewundene Strukturen, unabhängig von den Detektionsrezeptoren, die Detektionsrate für Zielstrukturen erhöhen. Es wurde festgestellt, dass durch die oben genannte Verdrehung:
1. beim Einsatz in einem blutführenden Gefäß die dreidimensionalen Strukturen eine Störung des laminaren Flusses erzeugen, dadurch Zielpartikel in den durch die zugewandten Flächen definierten Innenraum der Struktur gelangen und somit die Kontaktwahrscheinlichkeit von Strukturen aus dem Blut mit dem Funktionselement erhöht wird.
2. der strukturierte Funktionsabschnitt zwischen den zugewandten Flächen einen "Innenraum", bestehend aus möglichst großen, konkav geformten Bereichen mit strukturbedingt reduzierter Strömungsgeschwindigkeit bildet, wodurch die bei Kontakt der Zielstrukturen mit dem Funktionsabschnitt auftretende Scherspannung reduziert und dadurch die Anheftungswahrscheinlichkeit erhöht wird. Der definitionsgemäß gebildete spiralförmige Innenraum erhöht die Kontaktwahrscheinlichkeit zwischen den in den Innenbereich gelangten Zielstrukturen und dem Funktionsabschnitt, da diese Zielstrukturen infolge der parallel zur Venenwand verlaufenden Hauptströmungsrichtung mit hoher Wahrscheinlichkeit auf die Spiralstruktur auftreffen. Ferner bietet der definitionsgemäß gebildete Innenraum den gebunden Zielstrukturen einen Schutz vor Abrieb, wenn die Detektionsvorrichtung aus dem Körper entfernt wird.
3. die Oberfläche des Funktionselementes vergrößert wird.

Der Vorteil gegenüber beispielsweise einer zylindrischen Struktur ist die Durchmischung des im laminaren Strom befindlichen Blutes. Dies wird durch die bereits in der WO 2010/145824 A1 beschriebene Schraubenstruktur bewirkt und hat das Prinzip eines "statischen Mixers". Die Form des statischen Mixers bewirkt eine Durchmischung des vorbeiströmenden Blutes und damit eine erhöhte Wahrscheinlichkeit für Kollisionen von Zielstrukturen mit dem Funktionsabschnitt. Die durch die Schraubenform vergrößerte Oberfläche des Funktionsabschnitts erhöht ebenso die Wahrscheinlichkeit für Kontakte von Zielstrukturen.

Mögliche Ausführungsbeispiele für durch Torsion erzeugte Funktionsabschnitte haben beispielsweise dreieckige oder rechteckige Ausgangsquerschnitte. Der Funktionsabschnitt kann dabei als Dreikantstab oder als Flachstab vorzugsweise mit Naturkante ausgebildet sein und verdreht werden. Der mehreckige Querschnitt hat dabei an den Längsseiten eine hohle Form.

Der Funktionsabschnitt kann aus einem Metall, Kunststoff oder Keramik bestehen, vorzugsweise einem medizinischen Edelstahl oder einem optischen Polymer.

Der Funktionsabschnitt kann, wie schon in WO 2010/145824 A1 beschrieben, mit einer funktionalen, blutabweisenden Beschichtung versehen werden, an die die Detektionsrezeptoren gebunden werden können. Die Beschichtung kann aus einem synthetischen oder natürlichen Polymer oder Copolymer, welches bevorzugt Carboxylgruppen als funktionale Gruppen enthält, bestehen. Das Polymer ist vorzugsweise quervernetzt und kann über einen Haftvermittler an die Oberfläche des Funktionselements gebunden werden. Möglich ist auch die Kombination von zwei Polymeren, wie z.B. Polyelektrolyten, natürlichen oder synthetischen Ursprungs, welche eine (Monolayer) oder mehrere Schichten (Multilayer) bilden.

Im Rahmen dieser Erfindung wird die Detektionsvorrichtung vereinfachend lediglich als Vorrichtung bezeichnet.

Die Vorrichtung 1 basiert auf einem Draht mit 100 - 200 mm Länge. Am distalen Ende des Drahtes befindet sich der besagte Funktionsabschnitt 2, dessen Oberfläche mit Rezeptoren für Zielstrukturen funktionalisiert ist. Der Funktionsabschnitt 2 umfasst einen Durchmesser von bis zu maximal 3 mm, der minimale Durchmesser wird durch technische Möglichkeiten und die Materialeigenschaften bestimmt und sollte möglichst klein sein. Der Funktionsabschnitt hat eine Länge von 10 bis 200 mm. Der Funktionsabschnitt 2 besteht vorzugsweise aus einem Metall, vorzugsweise einem medizinischen Edelstahl, aus einem keramischen Material oder einem optischen oder sonstigen Polymer, vorzugsweise PMMA, und kann zur Ankopplung von Rezeptoren, mit einer blutabweisenden Schicht versehen werden, die ein Hydrogel oder eine funktionelles Polymer oder Copolymer sein kann.

### Ein Beispiel der Anmeldung (Fig. 1 (a) und (b))

Im ersten Ausführungsbeispiel gemäß Figur 1 basiert die Vorrichtung 1 auf einem Dreikant-Draht. Dabei weist der Funktionsabschnitt 2 eine Schraubenform auf, die durch Verschraubung des Ausgangsquerschnitts 3 in Gestalt eines gleichseitigen Dreiecks entlang der linearen Verschraubungsachse 4 erzeugt ist. Das gleichseitige Dreieck ist ein Beispiel eines dreizählig drehsymmetrischen Ausgangsquerschnitts 3, der durch Drehung um 120° und 240° um eine mit der Verschraubungsachse 4 zusammenfallende Drehachse 5 jeweils auf sich selbst abgebildet wird. Die Ecken des Ausgangsquerschnitts 3 können zusätzlich abgerundet werden, um eine Verletzungsgefahr zu reduzieren.

### Erstes Ausführungsbeispiel der Erfindung (Fig. 2 (a) und (b))

Im zweiten Ausführungsbeispiel gemäß Figur 2 weist der Draht abweichend vom ersten Ausführungsbeispiel gemäß Figur 1 an den Seiten des Dreiecks zusätzliche Einbuchtungen auf und ist an den Ecken abgerundet. Durch jede Vertiefung wird ein größerer Innenraum für die Bindung von Zielstrukturen geschaffen. Wie in Figur 2(b) zu erkennen ist, weist der Ausgangsquerschnitt 3 insgesamt drei Arme 6 auf, die sich jeweils ausgehend von der mit der Verschraubungsachse 4 zusammenfallenden Drehachse 5 in radialer Richtung erstrecken und eine Verdickung 7 sowie einen zwischen der Verdickung 7 und der Drehachse 5 angeordnete Verjüngung 8 aufweisen. Der Ausgangsquerschnitt 3 ist spiegelsymmetrisch und bezüglich der Drehachse 5 dreizählig drehsymmetrisch.

### Zweites Ausführungsbeispiel der Erfindung (Fig. 3 (a) und (b))

Das dritte Ausführungsbeispiel gemäß Figur 3 basiert auf der Verdrehung eines Flachdrahtes, der im Wesentlichen einen Ausgangsquerschnitt 3 in Form eines Knochens aufweist. Ein gerundeter Flachdraht kann durch Glattpressen eines Runddrahtes erzeugt werden, wodurch eine sogenannte Naturkante entsteht. Der Flachdraht kann flach, oder zusätzlich in der Mitte konkav eingebuchtet sein. Durch die zusätzliche Vertiefung wird ein größerer Innenraum für die Bindung von Zielstrukturen geschaffen. Der Ausgangsquerschnitt 3 ist doppelt spiegelsymmetrisch und bezüglich der Drehachse 5 zweizählig drehsymmetrisch. Die Krümmung der Arme erfolgt vorzugsweise in Verschraubungsrichtung.

### Weiteres Beispiel der Anmeldung (Fig. 4 (a) und (b))

Das Beispiel gemäß Figur 4 basiert im Wesentlichen auf einem dreiarmigen Ausgangsquerschnitt 3, dessen drei Arme 6 sich zunächst radial von der mit der Verschraubungsachse 4 zusammenfallenden Drehachse 4 erstrecken und am freien Ende bezüglich einer Radialen zur Drehachse 5 gekrümmt sind. Bei dieser Form ist der Innenraum der Funktionsfläche vergrößert und flüssigkeitsleitende Kanäle formen sich stärker aus. Dieser Ausgangsquerschnitt 3 ist aufgrund der einseitigen Krümmungen der Arme 6 nicht spiegelsymmetrisch, jedoch bezüglich der Drehachse 5 dreizählig drehsymmetrisch.

### Weitere Beispiele der Anmeldung (Fig. 5 bis 7)

In den Beispielen gemäß den Figuren 5-7 ist der Funktionsabschnitt 2 als Spirale, gerade oder spitz zulaufend, und mit unterschiedlicher Steigung ausgebildet. Die Vorrichtungen 1 sind jeweils auf einer rinnenförmigen Ablage dargestellt, die nicht zum Erfindungsgegenstand gehört.

Wenngleich die Vorrichtung hauptsächlich zur Anreicherung von Zielstrukturen im menschlichen Blutkreislauf (in vivo) ausgelegt und dimensioniert ist, versteht es sich von selbst, dass die Vorrichtung auch außerhalb des menschlichen Körpers (in vitro) verwendet werden kann.

### Bezugszeichenliste

- 1:: Vorrichtung
- 2:: Funktionsabschnitt
- 3:: Ausgangsquerschnitt
- 4:: Verschraubungsachse
- 5:: Drehachse
- 6:: Arm
- 7:: Verdickung
- 8:: Verjüngung

## Patentansprüche

1. Vorrichtung (1) zur in-vivo und/oder in-vitro Anreicherung von Zielstrukturen in einer Probeflüssigkeit, umfassend wenigstens einen Funktionsabschnitt (2), der mit Rezeptoren zur Anreicherung der Zielstrukturen bestückt ist, **dadurch gekennzeichnet, dass** der Funktionsabschnitt (2) eine Schraubenform aufweist, die durch Verschraubung eines symmetrischen Ausgangsquerschnitts (3) um eine Verschraubungsachse (4) entsteht, wobei der Ausgangsquerschnitt (3) ein konkaves Polygon ist.

2. Vorrichtung nach Anspruch 1, **dadurch gekennzeichnet, dass** der Ausgangsquerschnitt (3) spiegelsymmetrisch und/oder drehsymmetrisch, vorzugsweise mehrzählig drehsymmetrisch, bevorzugt zwei-, drei-, vier- oder fünfzählig drehsymmetrisch bezüglich einer Drehachse (5) ist.

3. Vorrichtung nach einem der vorangehenden Ansprüche, **dadurch gekennzeichnet, dass** der Ausgangsquerschnitt wenigstens einen Arm (6) aufweist, der sich vorzugsweise ausgehend von der Drehachse (5) entlang einer Linie erstreckt, wobei der Arm (6) bevorzugt wenigstens eine Verdickung (7) und/oder wenigstens eine Verjüngung (8) aufweist, wobei die Linie sich besonders bevorzugt radial zur Drehachse (5) erstreckt oder bezüglich einer Radialen zur Drehachse (5) gekrümmt ist.

4. Vorrichtung nach einem der vorangehenden Ansprüche, **dadurch gekennzeichnet, dass** die Verschraubungsachse (4) gleichzeitig die Drehachse (5) ist.

5. Vorrichtung nach einem der vorangehenden Ansprüche, **dadurch gekennzeichnet, dass** sich die Verschraubungsachse (4) innerhalb des Ausgangsquerschnitts (3) befindet, wobei die Verschraubungsachse (4) vorzugsweise durch den Flächenschwerpunkt des Ausgangsquerschnitts (3) verläuft.

6. Vorrichtung nach einem der vorangehenden Ansprüche mit Ausnahme des unmittelbar vorangehenden Anspruchs, **dadurch gekennzeichnet, dass** sich die Verschraubungsachse (4) außerhalb des Ausgangsquerschnitts (3) befindet.

7. Vorrichtung nach einem der vorangehenden Ansprüche, **dadurch gekennzeichnet, dass** die Schraubenform eine konstante Ganghöhe und/oder eine konstante Steigung und/oder einen konstanten Radius aufweist.

8. Verwendung der Vorrichtung (1) nach einem der vorangehenden Ansprüche zur in-vitro Anreicherung von Zielstrukturen in einer Probeflüssigkeit, umfassend die Schritte:
a. Einbringen des Funktionsabschnitts (2) in eine Probeflüssigkeit mit laminarer Strömung, vorzugsweise in einen in vitro Blutkreislauf, so dass sich die Verschraubungsachse (5) entlang oder im Wesentlichen entlang der Strömungsrichtung der Probeflüssigkeit erstreckt.
b. Anreicherung der Zielstrukturen in der Probeflüssigkeit an den Rezeptoren des Funktionsabschnitts (2).
c. Entnahme des Funktionsabschnitts (2) aus der Probeflüssigkeit.

## Claims

1. A device (1) for the in-vivo and/or in-vitro enrichment of target structures in a sample liquid, comprising at least one functional section (2) which is equipped with receptors for enriching the target structures, **characterized in that** the functional section (2) has a helical shape which is produced by screwing a symmetrical output cross-section (3) about a screwing axis (4), the output cross-section (3) being a concave polygon.

2. The device according to claim 1, **characterised in that** the output cross-section (3) is mirror-symmetrical and/or rotationally symmetrical, preferably multiple rotationally symmetrical, preferably two-, three-, four-or five-fold rotationally symmetrical with respect to an axis of rotation (5).

3. The device according to one of the preceding claims, **characterised in that** the output cross-section comprises at least one arm (6) which preferably extends from the axis of rotation (5) along a line, the arm (6) preferably comprising at least one thickening (7) and/or at least one tapering (8), the line extending particularly preferably radially to the axis of rotation (5) or being curved with respect to a radial to the axis of rotation (5).

4. The device according to one of the preceding claims, **characterised in that** the screwing axis (4) is at the same time the rotation axis (5).

5. The device according to any one of the preceding claims, **characterised in that** the screwing axis (4) is located within the exit cross-section (3), the screwing axis (4) preferably passing through the surface centre of gravity of the exit cross-section (3).

6. The device according to one of the preceding claims with the exception of the immediately preceding claim, **characterised in that** the screw connection axis (4) is located outside the outlet cross-section (3).

7. The device according to one of the preceding claims, **characterised in that** the screw shape has a constant pitch and/or a constant pitch and/or a constant radius.

8. The use of the device (1) according to any one of the preceding claims for in vitro enrichment of target structures in a sample liquid, comprising the steps:
a. Inserting the functional section (2) into a sample fluid with laminar flow, preferably into an in vitro blood circuit, so that the screw connection axis (5) extends along or substantially along the direction of flow of the sample fluid.
b. Enrichment of the target structures in the sample fluid at the receptors of the functional section (2).
c. Removal of the functional section (2) from the sample liquid.

## Revendications

1. Dispositif (1) d'enrichissement in-vivo et/ou in-vitro de structures cibles dans un échantillon liquide, comprenant au moins une section fonctionnelle (2) qui est équipée de récepteurs pour l'enrichissement des structures cibles, **caractérisé en ce que** la section fonctionnelle (2) a une forme hélicoïdale qui est produite par le vissage d'une section de sortie (3) symétrique autour d'un axe de vissage (4), la section de sortie (3) étant un polygone concave.

2. Dispositif selon la revendication 1, **caractérisé en ce que** la section transversale de sortie (3) est symétrique en miroir et/ou à symétrie de rotation, de préférence à symétrie de rotation multiple, de préférence à symétrie de rotation double, triple, quadruple ou quintuple par rapport à un axe de rotation (5).

3. Dispositif selon l'une des revendications précédentes, **caractérisé en ce que** la section de sortie comprend au moins un bras (6) qui s'étend de préférence à partir de l'axe de rotation (5) le long d'une ligne, le bras (6) comprenant de préférence au moins un épaississement (7) et/ou au moins un rétrécissement (8), la ligne s'étendant de manière particulièrement préférée radialement à l'axe de rotation (5) ou étant courbée par rapport à un radial à l'axe de rotation (5).

4. Dispositif selon l'une des revendications précédentes, **caractérisé en ce que** l'axe de vissage (4) est en même temps l'axe de rotation (5).

5. Dispositif selon l'une des revendications précédentes, **caractérisé en ce que** l'axe de vissage (4) est situé dans la section de sortie (3), l'axe de vissage (4) passant de préférence par le centre de gravité de la surface de la section de sortie (3).

6. Dispositif selon l'une des revendications précédentes à l'exception de la revendication immédiatement précédente, **caractérisé en ce que** l'axe de raccordement à vis (4) est situé à l'extérieur de la section de sortie (3).

7. Dispositif selon l'une des revendications précédentes, **caractérisé en ce que** la forme de la vis présente un pas constant et/ou un pas constant et/ou un rayon constant.

8. Utilisation du dispositif (1) selon l'une des revendications précédentes pour l'enrichissement in vitro de structures cibles dans un échantillon liquide, comprenant les étapes:
a. Insertion de la section fonctionnelle (2) dans un échantillon de fluide à écoulement laminaire, de préférence dans un circuit sanguin in vitro, de sorte que l'axe de raccordement à vis (5) s'étende le long ou sensiblement le long de la direction d'écoulement de l'échantillon de fluide.
b. Enrichissement des structures cibles dans le liquide de l'échantillon au niveau des récepteurs de la section fonctionnelle (2).
c. Retrait de la partie fonctionnelle (2) du liquide de l'échantillon.
